(19) Europäisches Patentamt
European Patent Office
Office européen des brevets

(11) Publication number: 0 183 184
A1

(12) # EUROPEAN PATENT APPLICATION

(21) Application number: 85114774.4

(22) Date of filing: 21.11.85

(51) Int. Cl.[4]: C 12 N 11/14
C 07 K 17/14, C 03 C 25/02

(30) Priority: 30.11.84 US 677107

(43) Date of publication of application: 04.06.86 Bulletin 86/23

(84) Designated Contracting States: BE CH DE FR GB IT LI NL

(71) Applicant: PPG INDUSTRIES, INC.
One PPG Place
Pittsburgh Pennsylvania 15272(US)

(72) Inventor: Beaver, Richard Paul
1524 Annette Avenue
Library Pennsylvania 15129(US)

(72) Inventor: Betts, Ronald Eugene
3950 Ingram Street
No. 13-204 San Diego California 92127(US)

(72) Inventor: Chiang, Lin-Chang
17129 West Bernardo Drive Apt. 203
San Diego California 92127(US)

(72) Inventor: Sanzero, George Valentine
4126 D Providence Road
Charlotte North Carolina 28211(US)

(74) Representative: Sternagel, Hans-Günther, Dr. et al,
Patentanwälte Dr. M. Hann Dr. H.-G. Sternagel
Marburger Strasse 38
D-6300 Giessen(DE)

(54) **Method of cultivating cellular biomaterial and glass fibers with cellular biomaterial or biomaterial.**

(57) Cellular biomaterial is immobilized on hollow and solid glass fibers, and biomaterial is immobilized on hollow glass fibers (10). The maximum population of cellular biomaterial or an inoculum of an amount of cellular biomaterial for reproduction to the maximum population can be immobilized on the glass fibers (10). The glass fibers (10) are formed and attenuated and contacted with the cellular biomaterial or biomaterial in the presence of activating fluids (14) and the immobilized material is maintained in an active state. In a containing vessel (12), the glass fibers (10) with immobilized cells from an inoculum cn be incubated at effective cell reproducing conditions to culture cellular biomaterial on the glass fibers (10) which can be frozen or used as hosts in producing biological and or biochemical products.

FIG. 1

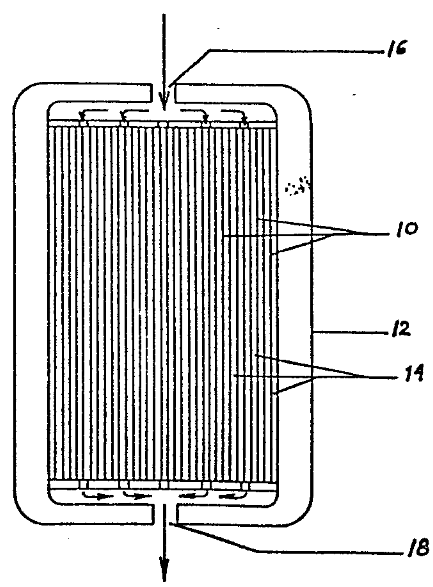


EP 0 183 184 A1

# METHOD OF CULTIVATING CELLULAR BIOMATERIAL AND GLASS FIBERS WITH CELLULAR BIOMATERIAL OR BIOMATERIAL

The present invention is directed to a method of producing glass fibers with biomaterial immobilized on the surfaces of the glass fibers and the products resulting from the process and the use of the products to culture cellular biomaterial.

With ever increasing interest, various chemical, biochemical and/or biological processes are being investigated, developed or used, where biomaterials are employed in various roles in the processes. Generally, the roles include modifying a starting material to produce a product such as through action as an initiator, catalyst and the like, and culturing various types of biological materials such as bacteria, yeasts, molds and animal cells. An example of a biomaterial for modifying starting material is enzymes, which are biochemical macromolecules with extraordinary properties of specificity in catalytic power to catalyze various chemical reactions. An example of culturing biomaterials is the bottle perfusion tissue culture technique.

The feasibility of both large scale commercial operations utilizing biomaterials in chemical and/or biochemical reactions and large scale commercial operations cultivating and/or harvesting biomaterials as products, intermediates, or the like is hampered for some biomaterials by economics and by limited stability of some biomaterials to mechanical and chemical changes in their environment. To circumvent these difficulties, the art has suggested immobilizing biomaterials such as enzymes on sundry supports. Such immobilization offers stability, reusability and recyclability of the immobilized enzymes. The supports mentioned in the art for immobilization of various biomaterial include polymeric materials such as fibers and also inorganic particles, beads and fibers for immobilizing enzymes. The various support materials all have their particular advantages and disadvantages for immobilization. The art has suggested that glass supports have not always found universal acceptance because of their limited capacity to bond or absorb biomaterials, whereas polymeric materials have a higher affinity for bonding or absorbing biomaterials. Several disadvantages of the numerous organic supports

include: their sensitivity to microbial attack, their lower thermal stability, which negates the possibility of sterilizing or autoclaving the polymeric supports, poor dimensional stability at elevated temperatures such that the support may be distorted or destroyed at such elevated temperatures, and not being inert to many chemicals including some strong sanitizing or oxidizing chemicals. On the other hand, the inorganic surfaces have excellent microbial resistance, excellent thermal stability to permit normal heat sterilization processes, excellent dimensional stability to avoid distortion and destruction at elevated temperatures, and an inertness to many sanitizing and oxidizing chemicals.

Realizing the advantages of inorganic surfaces, several researches have investigated and developed porous inorganic supports for immobilization of enzymes and bacteria, yeasts, and fungi. United States -A- 3,519,538 (Messing et al.) and 4,024,235 (Weetall et al.) and 4,286,061 disclose methods for bonding biomaterials directly to porous glass or ceramic beads. One method incorporates derivatives of silane compounds into the siliceous surfaces of the glass or ceramic beads and chemically coupling biologically or biochemically active molecules through the organic moiety of the organo silane. U.S. -A- 3,652,761 (Weetall) discloses a method whereby organic biological or immunological materials can be bonded to inorganic supports such as glass beads through the use of the organo silane material. Also U.S. -A-. 3,669,841 (Miller) discloses the use of a crosslinking agent to covalently attach enzymes to silylated siliceous materials. These siliceous materials include fibrous silica and silicates such as glass cloth, fibers, matting. In this method, a silane coupling agent in an inert solvent or aqueous solution is applied to the inorganic surface and crosslinked with the crosslinking agents such as glutaraldehyde.

In culturing cellular biomaterials, the traditional approaches use suspensions, liquid culture tanks for anchorage independent type cells and culture bottles for anchorage dependent

type cells. The former cells include yeasts in beer production and bacteria and the like, cells that grow in a liquid suspension unattached to any surface. The latter cells include mammalian cells that do not have thick cell walls like the former cell types and they must be anchored to a surface to multiply and grow. The surfaces of the culture bottles serves as the surface to which the latter type of cells become attached. Unfortunately, to attempt to provide large quantities of the latter cell type, the required number of culture bottles results in a space requirement problem. Numerous different types of polymeric beads and fibers recently have been suggested by the art for culturing cells, thereby obviating the use of culture bottles, but incurring disadvantages of non-heat sterilizability, lack of chemical inertness and sensitivity to microbial attack.

It is an object of the present invention to provide inorganic supports having immobilized biomaterials to allow for production of large quantities of biomaterials without the concomitant space requirements of culture bottles, where the supports provide large surface areas and high mechanical strength and good characteristics for use in chemical reactors, and the advantages of heat sterilizability, autoclavability, chemical inertness and resistance to microbial attack.

## SUMMARY OF THE INVENTION

The present invention accomplishes the aforementioned object and other objects gleaned from the following disclosure by producing glass fibers which have immobilized biomaterial or cellular biomaterial. The method for producing these glass fibers includes: forming solid or hollow glass fibers from a fiberizable glass forming batch composition, attenuating the fibers into chopped or continuous lengths, contacting the solid or hollow glass fibers with an activated fluid having the cellular biomaterial or the hollow glass fibers with an activated fluid having cellular biomaterial or biomaterial to immobilize the cellular biomaterial or biomaterial on the glass

fibers, and maintaining the activity of the cellular biomaterial or biomaterial immobilized on the glass fibers.

The glass fibers with immobilized biomaterial or cellular biomaterial have fiber diameters up to around 150 microns and discrete lengths ranging from those for chopped fibers as short as around (0.03 inch) 0.076 cm to those for continuous fibers. The fibers can be coated or uncoated and can be in the form of one or more individual fibers; a group or bundle of more than one fiber, hereinafter referred to as strand; a plurality of strands; yarn; mat; woven and unwoven fabric. To maintain the activity of the biomaterial or cellular biomaterial immobilized on the glass fibers, the glass fibers with the immobilized biomaterial or cellular biomaterial and activating fluids can be confined in a container having sufficient dimensions to house the fibers in contact with the activating fluid.

The method of cultivating cells utilizes cellular biomaterial immobilized on solid or hollow glass fibers so that the cells can multiply on the surfaces of the glass fibers. An effective inoculum of cellular biomaterial having a sufficient cell population and sufficient concentration of liquid activating fluid for multiplication of the cells is provided to the sterilized solid or hollow glass fibers. The inoculum and glass fibers are in a container which allows for incubation. The cells are immobilized on the glass fibers through adsorption or chemical bonding with linking agents on the glass fiber surfaces. The immobilized cells on the glass fibers are incubated in the presence of effective liquid and gaseous activating fluids and at activating conditions. The activity of the multiplied cells is maintained with activating fluids or through freezing.

## BRIEF DESCRIPTION OF THE DRAWING

FIGURE 1 is a longitudinal sectional view of the container with glass fibers having immobilized cellular biomaterial.

FIGURE 2 is a photograph of a hollow glass fiber with cells immobilized on its exterior and interior surfaces.

DETAILED DESCRIPTION OF THE INVENTION

In practicing the present invention in culturing cellular biomaterial, several additional advantages are realized.

It has recently been mentioned in the literature that glass spheres were not suitable for culturing anchorage dependent cells in stirred suspensions. The reason given was that the high density of the spheres required stirring speeds for the suspension which were not compatible with cell growth. It is also recognized in the art of culturing cells that in inoculating beads for multiplication of anchorage dependent beads that a sufficient cell population must be provided to inoculate each and every bead. Uninoculated beads usually will not participate in supporting multiplying cells since multiplying cells do not ordinarily bridge from one support surface to another separate support surface.

In culturing cells on discrete lengths of hollow glass fibers, it is believed without limiting the invention that the high surface area of both the exterior and interior surfaces allows for good cell growth while permitting adequate stirring in suspensions. Also it is believed without limiting the invention that the solid and hollow glass fibers with discrete lengths offer the advantage over spheres of using lower cell populations in inoculating the fibers for cell multiplication. The continuing surfaces of the discrete lengths of solid or hollow fibers can yield a higher surface area than individual spheres of the same radius. For the situations where the length of the solid fiber is at least twice its radius and where the length of hollow glass fibers is at least greater than its outer radius, the surface areas will be greater than that of a sphere with an equivalent radius. Hence, once the fiber is inoculated, the cells can multiply along its continuing surface area similar to inoculating several spheres. But the inoculation of the fiber would require fewer cells than needed to inoculate each sphere.

In the following disclosure and in the claims, the term "cellular biomaterial" refers to living cells which are anchorage dependent cells or anchorage independent cells. Nonexclusive examples of the cellular biomaterial include living or dead, eukaryotic or prokaryotic cells including primary, diploid, established and transformed cells such as bacterial, yeast, mold, fungi, plant, animal and mammalian cells, hybridomas, antibodies including monoclonal antibodies, antigens, immunoglobulins, antigen-antibody complexes and compliments, and other immunological materials, anchorage dependent animal cell lines such as cells from fish, reptiles, aves, including chickens and turkeys, mammals such as swine, sheep, rabbits, hamsters, mice, rats, primates, ferrets, dogs, cats, horses, goats and humans and other vertebrates and invertebrates, where such cell lines can be from tissues such as kidney, liver, lung, adrenal, heart, bone marrow, muscle, ovary, pancreas, glands, fibroblast epithelium, endothelium, amnion, lymphoid, leukemia, carcinoma, macrophage and cells from other tissues of these creatures.

The term "biomaterial products" refers to biological and/or biochemical substances that are produced from the cellular biomaterial, and nonexclusive examples include: monoclonal antibodies and other antibodies, and other immunological materials and viral, bacterial and like material produced using the cellular biomaterial as a medium.

In addition, when the glass fibers are hollow glass fibers, "biomaterial" can be immobilized in the lumen and on the exterior surface of the hollow glass fiber. The term "biomaterial" refers to substances which are either dead biological substances or biochemical substances, or materials which are capable of interacting with dead biological or biochemical substances or materials produced from a biochemical or dead biological substance, where these materials have chemical, biochemical or biological catalytic activity or production capability. Nonexclusive examples of the biomaterial include proteins, nucleoproteins, polynucleotides, polynucleosides;

lipoproteins, isozymes, lysozymes, hormones, endorphins, enkaphlins, organic or inorganic matter constituting substrates for enzymes, coenzymes, various enzymes, nonexclusive examples of which include: oxidoreductases, hydrolases, transferases, lyases, isomerses, ligases, etc. Examples of transferases are cretin phosphokinase, glycerol kinase, pyruvate kinase, hexhokinase, etc. Examples of isomerases are glucose phosphate isomerase, alanine isomerase, glucose isomerase, etc. Typical examples of ligase is glutathione synthetase. Examples of hydrolase are creatininase, cretinase, cephalosporinase, penicillinase, cephalosporin acylase, penicillin acylase, aminoacylase, urease, bromelein, papaine, chymotrupsin, trypsin, pepsin, galactosidase, glucosidase, amylase, phosphatase, cholesterolesterase, acetylchlolineesterease, phospholipase, lipase, etc. Examples of oxidoreductases are lipoxygenase, catalase, peroxidase, uricase, diaphorase, sarcosine oxidase, amine oxidase, amino acid oxidase, glutamic acid dehydrogenase, pyruvic acid oxidase, chlolineoxidase, galactoseoxidase, cholesteroloxidase, glucoseoxidase, 3-hydroxybutyrate dehydrogenase, glyclose-6-phosphase dehydrogenase, galactose dehydrogenase, lactate dehydrogenase, glycerol phosphaste dehydrogenase, glycerol dehydrogenase, alcohol dehydrogenase, viruses and and parts of cells such as cytoplasm, ectoplasm, endoplasm, karyolymp, karylosomes, nucleoli, chromatin, chondriosomes, mitrochondria, golgi bodies or trophospongium.

The biomaterial can be immobilized by adsorption, entrapment or chemical bonding. Also in the present invention in both the specification and the claims, the meaning of the term "activating fluids" refers to gases and liquids with materials to sustain the activity of the cellular biomaterial or biomaterial. For example, with cellular biomaterial, the liquids which may vary for different types of cellular biomaterial, have sufficient nutrients in a culture medium to encourage cell growth. For example, culture media can contain mixtures of amino acids, nucleic acids, carbon sources, choline, vitamins, inorganic salts for proper electrolyte balance,

blood serum supplements, antibodies and the like. Gaseous activating fluids supply gases necessary for cellular metabolism and multiplication. Another example is for enzymes and proteins and the like, where the solution has buffers and the like to assure the activity of the enzyme or protein.

The nonporous solid or nonporous hollow glass fibers of the present invention with the immobilized cellular biomaterial or biomaterial can be formed from any fiberizable, glass forming batch composition. Nonexclusive examples include: "E-glass", U.S. Patent 2,334,961, "621-glass", U.S. Patent 2,571,074 "A-glass", "C-glass" "S-glass", alkali metal borosilicate glasses and alkalimetal silicate glasses like those of U.S. Patents 2,106,744 and 3,972,720 and any more environmentally acceptable derivatives of these.

The glass batch compositions are melted in a direct melt glass batch melting furnace or in a marble preparation melting furnace at the temperature and time for the particular glass composition to obtain a fiberizable viscosity for the molten glass. Generally, the temperature ranges from about(2000°F) 1093°C to(3000°F) 1649°C for a time of around 1 to about 6 hours or longer. The molten glass is attenuated from the orifices of the bushing or spinnerets located around the bottom of the furnace or forehearth. The attenuation is conducted by mechanical means or thermal means by using heated fluid flow although mechanical attenuation is preferred. Where the fibers are formed and attenuated as hollow glass fibers, they can be formed and attenuated by methods described in U.S. Patents 3,268,313; 3,421,873; 3,510,393. Any other method of forming and attenuating the fibers as known by those skilled in the art can also be used. The fibers may be cooled, treated with a chemical protecting agent, i.e, a sizing composition, and gathered into one or more strands and chopped and collected or collected as continuous fibers or strands by any method known to those skilled in the art.

The glass fibers can have, and usually do have, a sizing composition applied to the glass fibers which protects the glass

fibers from interfilament abrasion in further processing steps. Any known sizing composition can be applied in art known quantities by any method known to those skilled in the art. The sizing composition is usually an aqueous composition with water soluble, dispersible or emulsifiable chemical agents that is placed on the glass fibers and remains on the glass fibers after the water and/or solvent is evaporated, but that may also be easily removable through solubility in a solvent, e.g. water. An example of a suitable water soluble chemical treatment is a cationic lubricant in water which is applied to the glass fibers. A suitable cationic lubricant includes Cation X® material, which is an alkyl imidazoline reaction product of tetraethylene pentamine and stearic acid. Other suitable material include textile softeners and cationic lubricants or agents generally known to those skilled in the art such as those disclosed in U.S.-A-4,002,445 (Graham).

After the sizing composition is applied to the glass fibers, the fibers are gathered into one or more strands, usually by means of a gathering shoe. In producing continuous glass fiber strands the glass fiber are wound onto a rotating drum-type winder having a forming tube to produce a forming package. The collet on which the forming package rides usually rotates at high speeds to collect the strand or strands into the forming package. Such speeds can be upward of 4,400 revolutions per minute which continues until the winder is slowed to a stop and the forming package is removed. An example of the forming process including sizing, gathering and collecting the glass fibers into a forming package, is disclosed in U.S.-A-4,071,339 (Griffiths) and U.S.-A- 4,049,411 (Long and Dent) where attenuation speeds of from about 2,000 to 20,000 feet per second are achieved.

The glass fibers and/or strands that are collected into the form of a multilayered package, either forming packages or roving packages or continuous fiberous or strand mats or batts may be removed from any packages by cutting or rewinding onto larger diameter drums

or can remain in the forms of packages, mat, or batt as do the chopped fibers or strands for contact with cellular biomaterials or biomaterials. Preferably the continuous strands are cut from one or more multilayered packages by making one or more cuts through the layers in a lengthwise manner extending parallel to the lengthwise axis of the package. The length of the cut glass fibers can be varied by varying the diameter of the forming package during winding of the glass fibers or by rewinding the glass fibers from the forming package onto a smaller or larger diameter package. The many layers of glass fibers which are removed from the package can be laid flat on a supporting surface. The supporting surface can be a plate or tray or moving conveyor belt. Generally, the discrete lengths of glass fibers obtained by this approach can range from about (1 inch) 2.54 cm to around (25 inches) 64 cm . Any other method for removing the glass fibers from the multilayered package can be employed. For example, the fibers can be unwound from the package and disposed as chopped strand or continuous strand onto another supporting surface or holder or rotating drum. Preferably, the discrete lengths of glass fibers can range from about (0.25 inch) 0.64 cm to around (70 inches) 180 cm and most preferably up to around (25 inches) 64 cm .

The solid or hollow glass fibers can be made chemically receptive for bonding with the cellular biomaterial or biomaterial. Any known chemical attaching agents for bonding the cellular biomaterial or biomaterial to glass surfaces can be used. Nonexclusive examples including linking agents, precipitating agents, crosslinking agents and substrates. For example, dual functional linking agents having an inorganic functional moiety and an organic functional moiety can be used. The inorganic moiety attaches to the internal and external surfaces of the glass fibers and has the organic moiety available for covalent bonding with any reactable organic moiety of the cellular biomaterial or biomaterial. Examples of these linking agents include organofunctional silane coupling agents, organofunctional titanate complexes and any other organofunctional

coupling agent known to those skilled in the art to be used with glass fibers like amino organosilane and silylaldehyde coupling agents. Also the linking agent can be a combination of coupling agents, where the organo functional coupling agent is reacted with an intermediate compound which is reactable with the cellular biomaterial or biomaterial. An example of this method includes reacting a material such as glutaraldehyde with an amino-organo functional silane or its hydrolysis products and either applying the reacted organo functional silane or its hydrolysis products to the glass fibers, or applying the amino-organo functional silane to the glass fibers and then treating the silynated glass fiber with the glutaraldehyde.

For the cellular biomaterials, additional linking agents that can be used include collagen, polymeric film forming materials such as acrylic polymers and vinyl acetate polymers and copolymers of these polymers with esters, ethylene and the like. The glass fibers are made chemically reactive by applying one or more chemical attaching agents to the glass fibers prior to or simultaneously with contact between the glass fibers and cellular biomaterial or biomaterial. One suitable method for linking the cellular biomaterial to the glass fibers involves the use of amineorgano silanes and collagen and lysine. The glass fibers are treated or sized with dimethylaminosilane like dimethylaminoethyltrimethoxy silane or dimethylamino propyltrimethoxy silane or their hydrolysis products or any other coupling agent. The collagen, through the epsilon amino group of lysine is attached to the silynated glass fiber by known chemical techniques. This material is now available for immobilization of the cellular biomaterial to the glass surface.

The glass fibers, whether solid or hollow, are containerized by placing a plurality of the glass fibers in any type of vessel having the necessary volume to hold the fibers and cellular biomaterial or biomaterial and any activating fluids. The fibers for a perfusion arrangement can be placed in a container in near or exact horizontal or vertical disposition to each other. Also, the fibers

can be maintained in a parallel or near parallel alignment for immobilization of the cellular biomaterial or biomaterial. For suspension type arrangement in using biomaterial or cellular biomaterial, the glass fibers of discrete length can be disposed in the suspension vessel by any method known in the art for disposing inorganic or organic beads. The packaging of the container is not great enough to prohibit the flow of liquid or gaseous fluid about the surfaces of the solid or hollow glass fibers. The packing density of the fibers in the container can range from about 0 to 100 percent. The fibers can be held in place in the container by various means, for example, by a screen or polymeric plug or by the frictional forces exerted by the packing density.

The application of the biomaterials to the hollow or chemically receptive hollow glass fibers is by contacting the biomaterial with the hollow or hollow chemically receptive glass fibers. The cellular biomaterial is contacted with the solid or hollow glass fibers or chemically receptive versions of these fibers in a similar manner, but the contacting is always in the presence of activating fluids. This contacting can occur, for instance, in a column process in which the glass fibers are packed in a column, or in a batch process in which the glass fibers are dispersed in a vessel or are submerged and surrounded by the cellular biomaterial or biomaterial in a vessel. As with the cellular biomaterial, the biomaterial is usually contacted with the glass fibers in the presence of an activating fluid which maintains the activity of the biomaterial. Any medium or fluid known in the art which does not deactivate the particular cellular biomaterial or biomaterial may be employed. The activating fluids for cellular biomaterials have components as previously described and are usually referred to as a culture medium. The medium composition, medium pH, medium flow rate, container dimensions, culture conditions, such as temperature, gaseous activating fluid composition and like factors all are dependent, to some degree, on the particular type of cellular biomaterial employed

and the desired process parameter and goals. Such factors can be determined readily by those skilled in the art. For example, known cell lines have been immobilized and cultured on polymer supports with the use of specific culture medium and conditions. These established mediums can also be used with the present invention. Also any method known in the art for conducting small scale compatibility testing of various culture mediums with specific types of cells can be used. For biomaterial, pH buffered aqueous solutions can be used. The solutions are adjusted to the various pH requirements for the biomaterial; for example, useful pH buffered aqueous solutions include: acetate buffers of pH 4 to 6, phosphate buffers of pH 6 to 8, borate buffers of pH 8 to 9, and these can be used for enzymes, proteins and nonliving cells or parts of cells with activity levels in these pH ranges.

The contacting of the hollow glass fibers and biomaterial for absorption or chemical bonding is performed at a temperature at which the biomaterial is not deactivated. This temperature is preferably at about 0°C to about 30°C. The amount of biomaterial used may be as much as that which saturates the absorption and/or chemical bonding capacity of the hollow glass fibers. The quantity absorbed on the hollow glass fibers can be determined by the presence or absence of a degree of activity of the biomaterial in an inert medium or by subjecting the immobilized biomaterial to biochemical activity assay as known to those skilled in the art or by any other method known to those skilled in the art. For instance, a total protein assay can be conducted or a percentage of activity of total protein can be determined.

The contacting of the solid or hollow glass fibers and the cellular biomaterial can be performed with widely varying cell populations. The cell population can be large enough to produce solid or hollow glass fibers with a monolayer of cells immobilized on a substantial portion of the external surface of the solid fibers or of the internal and external surfaces of the hollow fibers. For cells

having the ability to conduct multilayer diffusion, cell populations can be provided to result in multilayer cell immobilization on all or a substantial portion of the surfaces of the glass fibers. This immobilization is without any growth or multiplication of the cells to result in the monolayers or multilayers. Also the cell population can be as little as that necessary for an inoculum for cultivating cells by multiplication or reproduction on the available surfaces of the solid or hollow glass fibers to result in an increase in cell population to yield a monolayer or multiple layers of cells covering all or a substantial portion of the available surfaces of the fibers. The cell population in a cell line of the inoculum depends on the type of cells and plating efficiency of the cells. For some cells, the cell population of the inoculum can be on the order of around $10^5$ to about $10^6$ cells per milliliter of liquid activating fluid. One skilled in the art can easily determine the optimum population for both direct immobilization and immobilization through multiplication of the cells. Activating fluid with various populations within the given range can be started and observed for the most successful multiplication of cells. Reasonable variations in the population do not seriously slow the multiplication of the cells on the glass fiber. The best population for each cell type should be determined individually, where, in some instances, it may be necessary to operate outside the stated population range.

Other conditions for immobilization either direct or through cultivation of the cellular biomaterial include temperature and pH. The temperature of the liquid activating fluid with the cellular biomaterial contacting the glass fibers should be in the range of about 32°C to about 41°C and preferably 35 to 38°C when mammalian cells are to be immobilized. These temperatures are usual temperatures used in culturing cells of these animal origins. The pH of the activating fluid with the cellular biomaterial is controlled in the range of about 6.8 to about 7.2.

In cultivating the cellular biomaterial on solid or hollow glass fibers in a container vessel, the vessel is adapted for temperature and pH control and renewal of the activating fluid. The activating fluids are refurbished to provide for continued reproduction of the cellular biomaterial possibly at different ratios during the reproduction cycle until there is the maximum cell population on the glass fibers. Also the components of the activating fluids may vary during the reproduction cycle. These variations in reproductive rates and in components of the activating fluids are well understood by those skilled in the art and can be handled in accordance with art recognized practices. The vessel contains the glass fibers preferably in parallel or near parallel alignment and allows ingress flow of the activating fluids with the cell line and allows egress flow of depleted activating fluids and any cellular metabolic wastes. The vessel adequately seals to reduce the possibility of contaminations of and infections to the multiplying cellular biomaterial. The vessel should provide for convenient pH and temperature controls through sensors and appropriate control mechanisms. The temperature controls are like any known for incubator type vessels or jacketed vessels or vessels with immersion apparatus. The pH can be controlled through periodic additions of needed acids or bases or through modifying the carbon dioxide in the headspace gas by introducing air with modified carbon dioxide levels. Very often the cellular biomaterial themselves will maintain the pH of the stabilizing medium in the desired range. In newly started cultures, careful observance of pH is desirable to avoid rapid pH swings resulting in abrupt, metabolic changes to the cellular biomaterial.

In maintaining the activity of the biomaterial or cellular biomaterial immobilized on the glass fibers, the activating fluids can be refurbished at necessary intervals. Also the activating fluids can be the environment to permit the immobilized cellular biomaterial or biomaterial to be frozen or for the biomaterials to be freeze dried. Methods known by those skilled in the art for freezing cellular

biomaterial and biomaterial or for freeze drying biomaterial can be used. The freezing or freeze drying can be performed directly on the glass fibers without transfer to other surfaces. The glass fibers with immobilized materials are containerized and the materials are cooled to around -70°C at around -10°C/minute. Recovery of frozen cellular biomaterial is achieved by rapidly thawing the glass fibers in a 37°C water bath.

Figure 1 depicts a vessel suitable for culturing or using cellular biomaterials, or immobilizing or using biomaterials. The glass fibers 10 are vertically disposed in vessel 12, in parallel or near parallel alignment to each other. The vessel 12 also has liquid activating fluid 14 to maintain the activity of the cellular biomaterial or biomaterial in the interstices between and surrounding the vertically arranged fibers. The vessel has an inlet port 16 and an outlet port 18. Also the vessel can have a sufficient structure to allow for elevated or reduced temperatures and/or pressures within chemical engineering parameters. The glass fibers with immobilized cellular biomaterials or biomaterials are packed in vessel 12 with sufficient room between them to allow the stabilizing medium 14 to contact the surfaces of the glass fibers. Generally, the separation between fibers is on the order of magnitude of at least microns. In addition to the foregoing immobilization technique, means for retaining or confining the immobilized biomaterials and cellular biomaterials and the means for supplying activating fluids thereto and removing defficient activating fluids therefrom known to those skilled in the art can be used.

In culturing cellular biomaterials, the cellular biomaterial is harvested from the glass fibers by enzymatic and/or chemical methods. Nonexclusive examples include adding trypsin and/or chelating agents like salts of EDTA or of nitrolotriacetic acid to the liquid activating fluid. Effective amounts of these materials, possibly over a period of time, removes the cellular biomaterials from the glass fibers. For example, a sufficient amount of trypsin to

provide a 0.001 or higher percent solution in the activating fluid is effective in around one minute or longer. Enough EDTA to provide a 0.005 to 0.10 percent solution in the stabilizing medium can be effective. Another enzyme demobilization technique can be used for the cells immobilized on glass fibers with linking agents. The linking agents can be digested by the enzymes to release the cells. Other cellular demobilization techniques known in the art such as hypotonic treatment, cold treatment, sonication, lignocaine harvesting of macrophages and the like can be used. After the cells are demobilized from the glass fibers, they are removed from the vessel along with the activating fluids. The glass fibers can be sterilized and reused to culture a new crop of cellular biomaterial.

In addition, a sufficient population of immobilized cellular biomaterial, whether directly immobilized or grown on the surfaces of the glass fibers, can be utilized as media or hosts for production of biological, biochemical or chemical products. For instance, virus can be produced from the cellular biomaterial immobilized on the glass fibers in an activating fluid environment in a perfusion, suspension or container vessel. Chick embryo fibroblasts can be applied on the surfaces of solid and hollow glass fibers in the vessel and these fibroblasts can multiply under perfusion culture conditions. These fibroblasts can be transformed with Rous sarcoma virus (RSV). Also virus particles budding from cell surfaces or antibodies produced from cells on glass fibers in a vessel in response to antigens in the stabilizing fluid can be harvested in semi-batch or continuous manner. The activating fluid with the virus particles or antibodies is replaced with fresh stabilizing fluid in the vessel at periodic intervals that allow for sufficient concentrations of these materials to be produced. The virus particles or antibodies are then mechanically or chemically separated from the removed activating fluid.

Figure 2 is a photograph of hollow "E-glass" fiber with pig kidney cells 20 growing on the external and internal surfaces at 22

and 24 respectively. The dark C-shaped figure, 26, indicates a portion of an air bubble. The glass fiber had an OD of 100 microns and an ID of 60 microns. The photograph was taken by placing a culture dish containing fiber, cells and medium under a light microscope using 40x magnification.

PREFERRED EMBODIMENT OF THE INVENTION

It is preferred in cultivating cellular biomaterials such as whole mammalian cells to utilize both the external and internal surfaces of hollow glass fibers.

Preferably because of a convenient supply of glass forming batch material, the hollow glass fibers are prepared from a batch of "621-glass" so the glass fibers have the following composition:

7.2% $B_2O_3$ 54% $SiO_2$ 1% $Na_2O$ 14.3% $Al_2O_3$ 22.4 CaO

The batch material was mixed and melted at a temperature in the range of (2600°F) 1427°C to (2700°F) 1482°C for a melting time of 2 to 6 hours or longer and the forming temperature is in the range of (2200 to 2300°F) 1204°C to 1260°C. The fibers have an outside diameter of around 8 microns to about 150 microns and an internal diameter of about 5 microns to about 90 microns. These hollow fibers are formed in hollow tip bushings using an air flow so the K factor of the fibers is up to about 0.95. The K factor is the ratio of internal to external diameter. These fibers are attenuated with only the use of water sprays and without the addition of any sizing composition. The fibers are gathered into one or more strands and collected by a winder onto a forming tube. The winder attenuates the fibers at speeds in the range of around (200 ft/sec.) 60.96 m/sec. or more into the multilayered package.

The strands in the layers are cut from the package by one lengthwise cut running parallel to the longitudinal axis of the multilayered forming package and cutting across the strands to unfold the strands from the package. The strands from the package have a

length of about (0.25 inch) to about (63 inches) 0.64 to 160 cm . The glass fibers are preferably aligned in near parallel alignment to each other, when removed from the package and placed in a containing vessel. These fibers are contacted in a container with the liquid activating fluid with a cell line of mammalian cells. The contacting can be performed by simultaneous or sequential addition of the fibers and cell line with either being added first in the sequential addition. The conditions of contacting include a temperature of around 37°C to about 41°C. The vessel with the glass fibers and cell line is maintained at incubating conditions of temperature, pressure and time with any replacement of activating fluid, culture medium.

After a cell population is obtained where a majority of the external and internal surfaces of the hollow fibers are covered with a monolayer of cells, the glass fibers with immobilized cells are maintained in the surroundings of the culture medium. To maintain the activity of the cells with a constant culture medium having adequate nutritional values, two approaches can be taken. The first is to provide for a continuous ingress and egress of fresh culture medium. This refurbishes the nutritional value of the medium surrounding the cells, and it also removes cellular metabolic wastes. The second approach is to cool by refrigeration or actual freezing of the glass fibers with immobilized cells. The refrigeration to temperatures in the range of about -20°C to 3°C slows the metabolic rates of the cells, thereby, reducing their requirements for nutritional materials. Freezing the cells places them in a state of suspended animation. Freezing can be accomplished by liquid nitrogen and the glass fibers with cells can be frozen until needed for such uses as hosts for producing biological, biochemical or chemical products. If the cells are being cultured, the cells are preferably removed by an enzyme method as in trypsinolosis.

The present invention is further illustrated by the following nonlimiting example.

## EXAMPLES

Hollow glass fibers having fiber diameters of 15, 30, 70, and 100 microns were prepared having about 0.5 K factor. These fibers were prepared from a fiberizable glass forming batch composition resulting in glass fibers having a glass composition in weight percent of: 27 $B_2O_3$, 8 $Na_2O$, 31.4 $SiO_2$ and 3.6 $ZrO_2$. These glass fibers were prepared by melting glass forming batch calculated to yield the desired quantities of oxides at a temperature of (2600°F) 1427°C, for 3 hours in platinum crucibles. The melt was cooled and crushed into approximately (0.5 inch) 1.27 cm pieces and charged into a 4 tip hollow fiber bushing. The fibers were drawn on an (8") collet 20.32 cm rotating at 135 RPM after a 1 hour conditioning in the bushing melter at (2600°F) 1427°C to reduce the seed content of the glass. Air flow to the tips was set to result in a K factor of 0.5 for the various fiber diameters. These glass fibers were attenuated into continuous glass fibers without any sizing composition and with only the application of water and gathered into a multilayered package.

A cell line pf pig kidney cells was initiated in an activating fluid of a culture medium of Eagle's MEM minimum essential medium containing 10 percent serum from Gibco Laboratories tissue culture products ®330-1435 p 165, 1982 in culture dishes which contained the hollow glass fibers of various diameters were. The culture dishes were incubated for 3 days at 37°C with 5% carbon dioxide in air circulation through the incubator. The cell culture dishes were removed from the incubator and observed under 10x magnification for cell growth and attachment. The fibers had excellent cell attachment and a monolayer of cells was growing on the exterior and interior surfaces of the hollow glass fibers as shown in the photograph of Figure 2.

WE CLAIM:

1. A method of producing solid glass fibers having immobilized cellular biomaterial, characterized by:

a. forming glass fibers from fiberizable glass forming compositions, where the fibers are selected from the group consisting of solid and hollow fiber having filament diameters of up to about 150 microns to enable the glass fibers to be wound and where the hollow fibers have a K factor of greater than 0 and up to about 0.95,

b. attenuating the glass fibers,

c. contacting the glass fibers with an activated fluid having cellular biomaterial under effective activating conditions, and

d. maintaining the activity of the immobilized cellular biomaterial.

2. Method of Claim 1, wherein the formed glass fibers are gathered into one or more strands, and collected as continuous or chopped glass fiber strands before contact with the activating fluid of cellular biomaterial.

3. Method of Claims 1-2, which includes sterilizing the formed glass fibers for contact with the activated fluid of cellular biomaterial in a sterilized environment.

4. Method of Claims 1-3, which includes treating the glass fibers with linking agents before the fibers are contacted with the activating fluid having cellular biomaterial.

5. Method of Claims 1-4, wherein maintaining the activity of the immobilized cellular biomaterial includes continuously removing a portion of the activating fluid which has been in contact with the

glass fibers for a period of time and replacing it with fresh activating fluid.

6. Method of Claims 1-4, wherein the maintaining of the activity of the immobilized cellular biomaterial occurs at ambient or subatmospheric temperature conditions including maintaining in a frozen condition.

7. Method of Claims 1-6, wherein the activated fluid having cellular biomaterial is effective for initiating multiplication of the biomaterial on the glass fibers.

8. Method of Claim 1-7, which includes applying a sizing composition to the glass fibers before the fibers are gathered.

9. Method of Claim 8, wherein the sizing composition is selected from the group consisting of aqueous soluble, dispersible or emulsifiable: nonionic, cationic lubricants; polymeric film forming polymers; coupling agents; nonionic, cationic emulsifiers; and mixtures thereof.

10. Method of Claims 2-9, which includes removing the glass fiber strands from the collection to maintain at least near parallel alignment of the glass fiber strands.

11. Method of Claims 2-10, which includes collecting the one or more glass fiber strands on a winder into a cylindrical package having a plurality of layers of continuous glass fiber strands, and the layers of strands are removed from the cylindrical package by cutting one or more longitudinal cuts parallel to the longitudinal axis of the package.

12. Method of Claim 11, which includes rewinding the glass fiber strands onto smaller or larger diameter packages to facilitate procurement of longer or shorter length glass fiber strands upon cutting the strands from the package.

13. Method of Claims 11-12, wherein the cut layers of glass fiber strands have a length ranging from less than one inch to around 63 inches and are laid in at least near parallel alignment for contacting with the activating fluid of cellular biomaterial.

14. Solid glass fibers with cellular biomaterial produced according to the methods of claims 1-13.

15. An article having biomaterial or cellular biomaterial with substantial activity immobilized on a support, characterized by:

a. a container means to retain an activating environment for biomaterial or cellular biomaterial, and

b. one or more nonporous hollow glass fibers having filament diameters up to 150 microns and a K factor of up to around 0.95 and a length from less than an inch to continuous lengths disposed in the container means, and

c. biomaterial or cellular biomaterial immobilized on the exterior surface of the glass fibers and in the lumen of the glass fibers by absorption or chemical bonding with a linking agent on the surface of the glass fibers, where the biomaterial or cellular biomaterial have substantial activity, and

d. an effective activating environment surrounding the glass fibers in the container means to maintain the activity of the biomaterial or cellular biomaterial.

16. A method of harvesting cellular biomaterials, characterized by:

a. providing an effective inoculum of cellular biomaterial having a sufficient cell population and a sufficient concentration of liquid activating fluid for multiplication of the cells to one or more sterilized glass fibers in a container means for immobilization of a sufficient number of cells on the glass fiber to allow for multiplication of the cells on the surfaces of the glass fibers,

b. incubating the cells immobilized on the glass fibers in the presence of effective liquid and gaseous activating fluids and at effective activating conditions to have the cells multiply on substantial portions of the surfaces of the glass fibers,

c. maintaining effective activating fluids surrounding the multiplying cells immobilized on the glass fibers to retain substantial activity of the immobilized cellular biomaterial.

17. Method of Claim 16, wherein the glass fibers are hollow glass fibers and the cellular biomaterial is grown on the exterior and interior surfaces of the glass fibers.

18. Method of Claims 16-17, which includes aligning the glass fibers in nearly parallel alignment in the container means.

19. Method of Claims 16-18, which includes removing the immobilized cells from the surface of the glass fibers and collecting the cells in activating fluids.

20. Method of Claims 16-18, which includes adding to the grown cellular biomaterial immobilized on the glass fibers in an

activating environment a biomaterial which stimulates the immobilized biomaterial to act as a host and produce product biomaterial.

21. Method of Claims 16-20, which includes removing the activating fluids and freezing the immobilized cellular biomaterial to maintain the activity of the immobilized cellular biomaterial.

22. Method of Claims 16-20 which includes treating the glass fibers with organosilane coupling agents to yield glass fibers having positive charges, collagen and lysine.

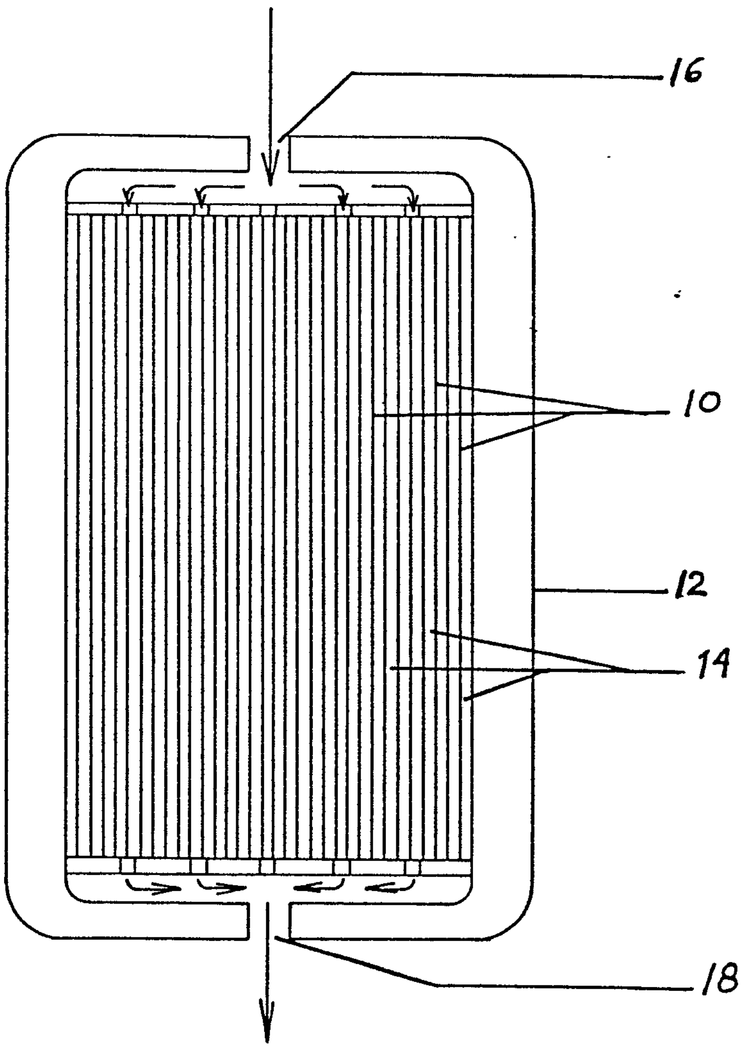


FIG. 1

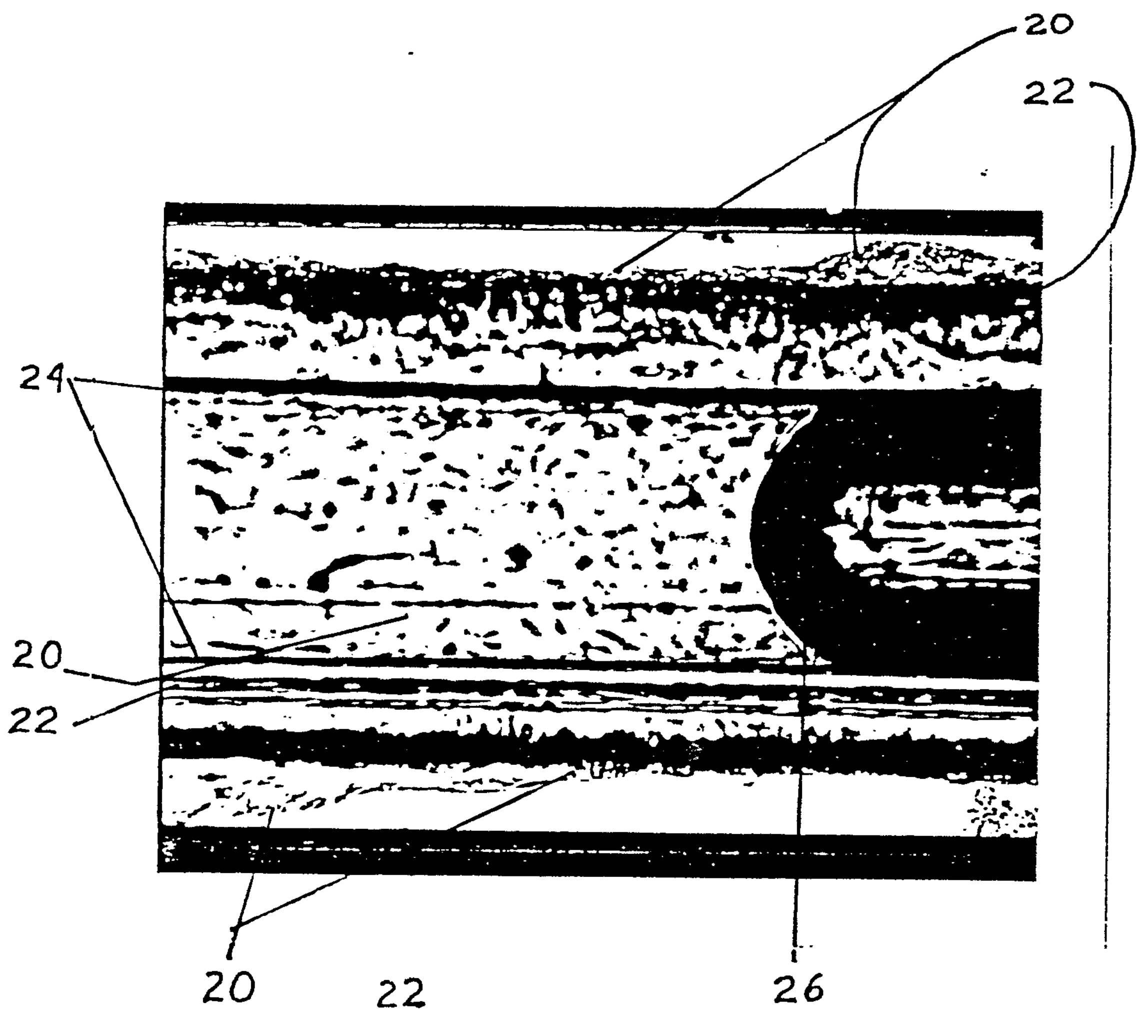


FIG. 2

European Patent Office

**EUROPEAN SEARCH REPORT**

0183184
Application number

EP 85114774.4

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| A | DE - A1 - 3 033 076 (BISSE) * Page 4, lines 26-30; page 9, line 29 - page 10, line 1; claims 1,3 * | 1,4,6, 15,22 | C 12 N 11/14<br>C 07 K 17/14<br>C 03 C 25/02 |
| A | DD - A - 141 035 (MEDIZINISCHE AKADEMIE MAGDEBURG) * Totality * | 1 | |
| A | US - A - 4 477 524 (BROWN) * Column 1, lines 15-35; column 7, lines 52-64; column 8, lines 35-68 * | 1,2,4, 8-12 | |
| A | US - A - 4 435 474 (DAS) * Column 1, lines 19-31; claims 1-9 * | 1,2,4, 8-12 | TECHNICAL FIELDS SEARCHED (Int Cl 4)<br>C 12 N<br>C 07 K<br>C 03 C<br>C 03 B<br>B 01 D |

The present search report has been drawn up for all claims

| Place of search | Date of completion of the search | Examiner |
|---|---|---|
| VIENNA | 12-02-1986 | BECKER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82